(19) Europäisches Patentamt
European Patent Office
Office européen des brevets

(11) Veröffentlichungsnummer: **0 062 608**
**B1**

(12) **EUROPÄISCHE PATENTSCHRIFT**

(45) Veröffentlichungstag der Patentschrift: **10.07.85**

(21) Anmeldenummer: **82810135.2**

(22) Anmeldetag: **25.03.82**

(51) Int. Cl.⁴: **C 07 C 121/70**, C 07 C 69/65, C 07 C 69/618, C 07 C 67/347, C 07 C 25/28, C 07 D 295/18

(54) **4-Halogenstilbenderivate und Verfahren zu deren Herstellung.**

(30) Priorität: **31.03.81 CH 2180/81**

(43) Veröffentlichungstag der Anmeldung: **13.10.82 Patentblatt 82/41**

(45) Bekanntmachung des Hinweises auf die Patenterteilung: **10.07.85 Patentblatt 85/28**

(84) Benannte Vertragsstaaten: **CH DE FR GB IT LI**

(56) Entgegenhaltungen:
**AT - B - 219 035**
**DE - A - 1 806 098**
**DE - A - 2 602 750**
**DE - B - 1 914 601**

(73) Patentinhaber: **CIBA-GEIGY AG, Postfach, CH-4002 Basel (CH)**

(72) Erfinder: **Reinehr, Dieter, Dr., Wolfsheule 10, D-7842 Kandern (DE)**
Erfinder: **Spencer, Alwyn, Dr., Schützengraben 15, CH-4051 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft neue 4-Halogenstilbenderivate und Verfahren zu deren Herstellung. Die neuen 4-Halogenstilbenderivate stellen wertvolle Zwischenprodukte zur Herstellung von optischen Aufhellern des Divinylstilbentyps dar.

Optische Aufheller des Divinylstilbentyps sind z. B. in der US-Patentschrift 4 108 887 beschrieben. Deren Herstellung ist jedoch, vor allem wenn es sich um asymmetrische Verbindungen handelt, ziemlich aufwendig. Auch sind die dazu benötigten Ausgangsprodukte, z. B. die Aldehyde, zum Teil nur schwer zugänglich. Aus der AT-B-219 035 ist die Herstellung von 1,4-Bis-(styryl)-benzolen nach Wittig-Horner bekannt. Bei dieser Herstellung fallen bedeutende Mengen phosphorhaltiger Nebenprodukte an.

Gegenstand der Erfindung sind neue 4-Halogenstilbenderivate der Formel I

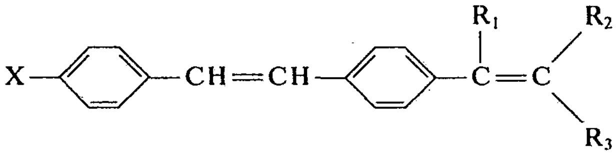

(I)

worin

X Brom oder Jod,
$R_1$ Wasserstoff, $C_{1-6}$-Alkyl oder eine nicht chromophore veresterte Carboxylgruppe,
$R_2$ unsubstituiertes oder nicht chromophor substituiertes $C_{1-6}$-Alkyl, nicht chromophor substituiertes $C_{2-4}$-Alkenyl, Phenyl oder durch Fluor, Chlor, $C_{1-4}$-Alkoxy, $C_{2-6}$-Alkoxyalkoxy, Di-($C_{1-4}$-alkyl)-amino oder $C_{1-4}$-Alkoxycarbonyl substituiertes Phenyl, oder einen nicht chromophoren Substituenten zweiter Ordnung und
$R_3$ Wasserstoff oder unsubstituiertes oder nicht chromophor substituiertes $C_{1-6}$-Alkyl oder nicht chromophor substituiertes $C_{2-4}$-Alkenyl bedeuten.

Die Verbindungen der Formel I lassen sich auf sehr einfache Weise und unter Verwendung von leicht zugänglichen Ausgangsprodukten dadurch herstellen, daß man entweder

a) eine Verbindung der Formel IIa

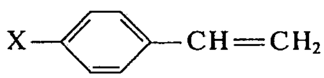

(IIa)

in Gegenwart einer Base und unter Zusatz von Palladium-Metall oder von Palladiumverbindungen, die unter den Reaktionsbedingungen phosphorfreie labile Palladium-(O)-Verbindungen bilden, als Katalysator, mit einer Verbindung der Formel IIIa

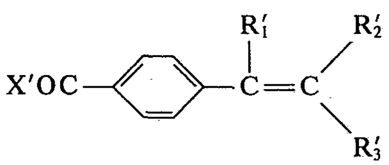

(IIIa)

oder

b) eine Verbindung der Formel IIb

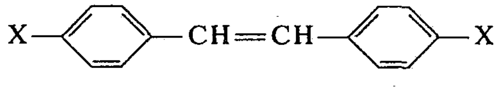

(IIb)

in Gegenwart einer Base mit einer arsen- oder phosphorhaltigen Palladiumverbindung mit einer Verbindung der Formel IIIb

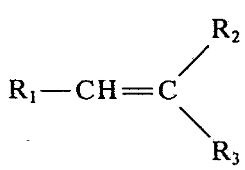

(IIIb)

umsetzt, wobei X, $R_1$, $R_2$ und $R_3$ die unter Formel I angegebene Bedeutung haben, X' Chlor, Brom oder Jod, bevorzugt Chlor, darstellt und $R'_1$, $R'_2$ und $R'_3$ dieselbe Bedeutung wie $R_1$, $R_2$ und $R_3$ haben können, jedoch frei von Substituenten sind, die zur Reaktion mit Gruppen X'OC— befähigt sind, wie

$-OH-$, $-NH_2-$, $-NH-$ oder COOH-Gruppen.

Die Herstellung gemäß Verfahrensvariante a) ist bevorzugt.

Die in den Verbindungen der Formel I vorkommenden Substituenten sind im allgemeinen solche, wie sie im Gebiet der optischen Aufheller üblich sind.

X stellt vorzugsweise Brom dar. Alkylgruppen $R_1$, $R_2$ und $R_3$ können geradkettig oder verzweigt sein und weisen 1–6 und insbesondere 1 oder 2 C-Atome auf. Alkenylgruppen $R_2$ und $R_3$ enthalten 2 bis 4, vorzugsweise 3 C-Atome. Als Beispiele derartiger Alkyl- und Alkenylgruppen seien erwähnt: die Methyl-, Äthyl-, n-Propyl-, Isopropyl-, n-Butyl-, tert.-Butyl, n-Pentyl-, n-Hexyl-, Vinyl- und Allylgruppe.

Als nicht chromophore Substituenten an definitionsgemäßen Phenyl-, Alkyl- oder Alkenylgruppen kommen z. B. Alkoxy-, Alkoxyalkoxy-, Dialkylamino- oder Alkoxycarbonylgruppen, ferner Halogenatome, wie Fluor oder Chlor, oder unsubstituiertes oder nicht chromophor substituiertes Phenyl in Betracht, z. B. gegebenenfalls durch Alkyl, Alkoxy, Halogen, Cyano, eine Carbonsäureester- oder -amidgruppe oder durch einen Carbonsäureacylrest substituiertes Phenyl. Alkoxy- oder Alkylteile in den genannten Substituenten enthalten 1 bis 4 und besonders 1 oder 2 C-Atome. Alkenylgruppen $R_2$ und $R_3$ sind bevorzugt unsubstituiert. Alkoxyalkoxy-Substituenten weisen 2–6 und insbesondere 4–6 C-Atome auf. Alkylenbrückenglieder in Alkoxyalkoxysubstituenten sind vorteilhaft Äthylen, Propylen-1,2, Butylen-1,2 oder Butylen-2,3, wovon jedoch Äthylen bevorzugt ist.

Nicht chromophore Substituenten zweiter Ordnung sind elektronenanziehende Substituenten, die den Verbindungen der Formel I keine Farbe verleihen, was deren Verwendung zur Herstellung von optischen Aufhellern beeinträchtigen würde. Als Beispiele seien genannt: Acylreste organischer nicht chromophorer Carbonsäuren, die Cyano- und Trifluormethylgruppe, gegebenenfalls substituiertes Phenylalkyl, Carbonsäureester- und -amidgruppen. Die im Molekül vorkommenden Estergruppen, besonders die nicht chromophoren veresterten Carboxylgruppen sind z. B. gegebenenfalls alkylsubstituierte Carbonsäurecycloalkylestergruppen oder Carbonsäurealkylestergruppen, wobei das Alkyl durch Alkoxy- oder Alkoxyalkoxygruppen der oben erwähnten Art substituiert sein kann; ferner gegebenenfalls substituierte Carbonsäurephenylester-oder -phenylalkylestergruppen, wobei das Phenyl wie oben angegeben substituiert sein kann.

Carbonsäureamidgruppen können substituiert oder unsubstituiert sein. Für die Verfahrensvariante a) kommen nur cyclische oder disubstituierte Carbonsäureamidgruppen in Betracht. Geeignete Substituenten sind z. B. Alkyl-, Alkoxyalkyl-, Dialkylaminoalkyl-, Cycloalkyl- und gegebenenfalls substituierte Phenyl- oder Phenylalkylgruppen.

Bevorzugt sind Verbindungen der Formel I, worin X Brom, $R_1$ Wasserstoff, $C_{1-6}$-Alkyl oder $-COOR'''$, bevorzugt Wasserstoff oder $C_{1-4}$-Alkyl und insbesondere Wasserstoff oder Methyl, $R_2$ unsubstituiertes oder durch $C_{1-4}$-Alkoxy oder $C_{2-5}$-Alkoxycarbonyl substituiertes $C_{1-6}$-Alkyl, besonders $C_{2-4}$-Alkenyl, $-CN$, $-CF_3$, Phenyl oder eine Gruppe

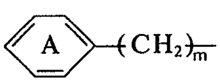

$-CO-R$, $-CO-NR'R''$ oder $-COOR'''$, mit Vorteil $C_{2-4}$-Alkenyl und vor allem $-CN$, Phenyl, $-CONR'R''$, $-COOR'''$ oder $-CO-R$, insbesondere $-CN$, Phenyl oder $-COOR'''$, $R_3$ Wasserstoff oder unsubstituiertes oder durch $C_{1-4}$-Alkoxy oder $C_{2-5}$-Alkoxycarbonyl substituiertes $C_{1-6}$-Alkyl, besonders Wasserstoff, $C_{1-4}$-Alkyl oder $-CH_2COO-C_{1-4}$-Alkyl und ganz besonders bevorzugt Wasserstoff, Methyl oder $-CH_2COOCH_3$ oder $-CH_2COOC_2H_5$, R Methyl, unsubstituiertes oder durch $C_{1-4}$-Alkoxy, $C_{4-6}$-Alkoxyalkoxy substituiertes $C_{2-6}$-Alkyl, einen Rest der Formel

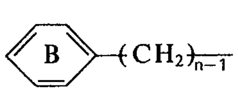

oder Naphthyl, besonders Methyl, unsubstituiertes oder durch $C_{1-4}$-Alkoxy oder $C_{4-6}$-Alkoxyalkoxy substituiertes $C_{2-6}$-Alkyl, den Rest der Formel

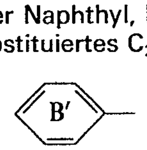

oder Naphthyl und ganz besonders unsubstituiertes oder durch Chlor, Methyl oder Methoxy monosubstituiertes Phenyl, unsubstituiertes $C_{1-6}$-Alkyl, vorzugsweise $C_{1-4}$-Alkyl und vor allem Methyl, R' Wasserstoff, Methyl, unsubstituiertes oder durch $C_{1-4}$-Alkoxy, $C_{4-6}$-Alkoxyalkoxy oder Di-($C_{1-4}$-alkyl)-amino substituiertes $C_{2-6}$-Alkyl, unsubstituiertes oder methyl- oder äthylsubstituiertes Cyclohexyl oder einen Rest der Formel

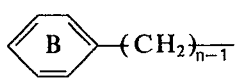

besonders Methyl oder Äthyl, oder zusammen mit R'' $-(CH_2)_4-$ oder $-(CH_2)_5-$, R'' Wasserstoff, Methyl oder unsubstituiertes oder $C_{1-4}$-Alkoxy oder $C_{4-6}$-Alkoxyalkoxy substituiertes $C_{2-6}$-Alkyl, besonders Methyl oder Äthyl, oder zusammen mit R' $-(CH_2)_4-$ oder $-(CH_2)_5-$, R''' Methyl, unsubstituiertes oder durch $C_{1-4}$-Alkoxy oder $C_{3-6}$-Alkoxyalkoxy substituiertes $C_{2-6}$-Alkyl, unsubstituiertes oder methyl- und/oder äthylsubstituiertes Cyclohexyl oder einen Rest der Formel

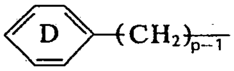

und besonders Methyl oder unsubstituiertes oder durch $C_{1-4}$-Alkoxy oder $C_{3-6}$-Alkoxyalkoxy substituiertes $C_{2-6}$-Alkyl, insbesondere Alkyl mit 1–6 und vor allem 1–4 C-Atomen und ganz besonders Methyl oder Äthyl, m die Zahl 1 oder 2, vorzugsweise 1, und n und p unabhängig voneinander die Zahl 1, 2 oder 3 und vorzugsweise 2 und insbesondere 1 bedeuten.

Dabei bedeutet vorzugsweise mindestens eines von $R_1$ und $R_3$ Wasserstoff, und die aromatischen Ringe A, B und D können durch $C_{1-3}$-Alkyl und/oder Halogen, besonders Chlor, mono- oder disubstituiert sein. Wenn n und p je die Zahl 1 bedeuten, können die aromatischen Ringe B und D unsubstituiert oder durch $C_{1-3}$-Alkyl und/oder Halogen trisubstituiert oder durch $C_{1-3}$-Alkoxy mono- oder disubstituiert sein. Der Ring B' ist unsubstituiert oder durch Halogen, besonders Chlor, $C_{1-3}$-Alkyl und/oder $C_{1-3}$-Alkoxy substituiert. Vorzugsweise sind die erwähnten Ringe unsubstituiert. R' und R'' haben vorzugsweise dieselbe Bedeutung oder stellen zusammen $-(CH_2)_4-$ oder $-(CH_2)_5-$ dar.

Des weiteren bevorzugt sind Verbindungen der Formel I, worin X Brom, $R_1$ Wasserstoff oder $C_{1-6}$-Alkyl, $R_2$ $C_{2-4}$-Alkenyl, –CN, Phenyl, –CONR'R'', –COOR''' oder –CO–R und $R_3$ Wasserstoff, $C_{1-4}$-Alkyl oder $-CH_2COO-C_{1-4}$-Alkyl bedeuten, wobei mindestens eines von $R_1$ und $R_3$ Wasserstoff ist und R, R', R'' und R''' die oben angegebene Bedeutung haben. R stellt dabei bevorzugt $C_{1-6}$-Alkyl oder unsubstituiertes oder durch Halogen, besonders Chlor, $C_{1-3}$-Alkyl oder $C_{1-3}$-Alkoxy substituiertes Phenyl, insbesondere unsubstituiertes $C_{1-4}$-Alkyl oder unsubstituiertes oder durch Chlor, Methyl oder Methoxy monosubstituiertes Phenyl dar. R' und R'' sind bevorzugt je Methyl oder Äthyl oder stellen zusammen $-(CH_2)_5-$ dar. R''' stellt vorzugsweise Methyl oder unsubstituiertes oder durch $C_{1-4}$-Alkoxy oder $C_{3-6}$-Alkoxyalkoxy substituiertes $C_{2-6}$-Alkyl, insbesondere unsubstituiertes $C_{1-4}$-Alkyl dar.

Besonders bevorzugt sind Verbindungen der Formel I, worin X Brom, $R_1$ Methyl und vor allem Wasserstoff, $R_2$ Phenyl, –CN,

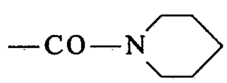

oder –COOR''', R''' unsubstituiertes $C_{1-4}$-Alkyl, vor allem Methyl oder Äthyl, und $R_3$ Wasserstoff, Methyl, $-CH_2COOCH_3$ oder $-CH_2COOC_2H_5$ bedeuten, wobei mindestens eines von $R_1$ und $R_3$ Wasserstoff ist.

Von ganz besonderem Interesse sind Verbindungen der Formel I, worin X Brom, $R_1$ und $R_3$ Wasserstoff und $R_2$ –CN oder $-COOC_2H_5$ bedeuten.

Die Katalysatoren sowie die Verbindungen der Formeln IIa, IIb, IIIa und IIIb sind bekannt oder können in an sich bekannter Weise hergestellt werden.

Gegenstand der Erfindung sind auch gewisse neue Ausgangsprodukte der Formeln IIIa, nämlich die Verbindungen der Formeln

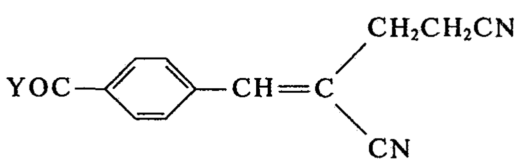

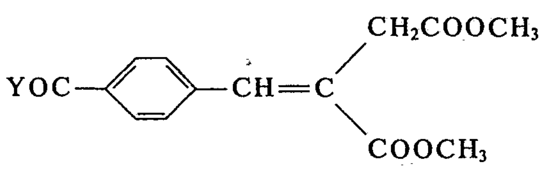

und

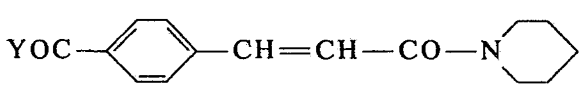

worin Y –OH oder –Cl darstellt. Diese wurden speziell für die Herstellung der erfindungsgemäßen Verbindungen der Formel I entwickelt.

Bezüglich der Herstellung von Verbindungen der Formel IIIb vgl. z. B. Houben-Weyl, Bd. 5/1b (1972). Die Verbindungen der Formel IIIa können z. B. durch Umsetzung von 4-Brombenzoesäure mit Verbindungen $HC(R_1)=C(R_2)(R_3)$ gemäß J. Org. Chem. 42, 3903 (1977) und anschließende Überführung des Reaktionsprodukts in definitionsgemäße Säurehalogenide hergestellt werden.

Die Verbindungen der Formel IIa und IIIa bzw. IIb und IIIb werden im allgemeinen in stöchiometrischer Menge eingesetzt. Es ist aber auch möglich, einen Überschuß an Verbindung der Formel IIa oder IIb, z. B. einen bis etwa 5fachen molaren Überschuß an Verbindung der Formel IIa bzw. IIb, zu verwenden.

Als definitionsgemäße Palladiumverbindungen für die Umsetzung gemäß Verfahrensvariante a) können — neben Palladium-Metall — z. B. Verbindungen der Formel IV

$$M^y[PdL_{n_1}]^x \qquad (IV)$$

eingesetzt werden, worin $n_1$ eine ganze Zahl von 2 bis 4, x $2^{\oplus}$ bis $2^{\ominus}$, $y = -(x)$, M, bei x ungleich 0, ein Gegenion und die L gleiche oder verschiedene phosphorfreie Liganden darstellen, wie z. B. Cl, Br, J, $-CN$, $-NO_3$, $C_{1-12}$-Alkyl$-COO$, $CH_3COCHCOCH_3$, $NH_3$, 2,2'-Bipyridyl, o-Phenanthrolin, $OS(CH_3)_2$ oder $-NC-$Phenyl. Geeignete Verbindungen der Formel IV sind beispielsweise $PdCl_2$, $PdBr_2$, $Pd(CN)_2$, $Pd(NO_3)_2$, $Pd(O_2C-C_{1-12}$-Alkyl), besonders $Pd(OOCCH_3)_2$, $Pd(CH_3COCHCOCH_3)_2$, $[Pd(NH_3)_4]Cl_2$, $[PdCl_4]Na_2$, $Pd(OOCCH_3)_2$(2,2'-Bipyridyl), $Pd(OOCCH_3)_2$-(o-Phenanthrolin), $PdCl_2[OS(CH_3)_2]_2$ und $PdCl_2(NC-\text{Phenyl})_2$.

Außer den oben genannten Verbindungen können für die Verfahrensvariante a) auch Palladiumverbindungen anderer Oxidationsstufen eingesetzt werden, z. B. Bis-(Dibenzylidenaceton)Palladium(O) und Bis-(Isonitril)Palladium(O)-Verbindungen. Als Beispiele solcher Isonitrile seien genannt: Bis-(Cyclohexylisonitril)Palladium(O), Bis-(Isopropylisonitril)Palladium(O), Bis-(tert.-Butylisonitril)Palladium(O), Bis-(p-Tolylisonitril)Palladium(O), Bis-(Phenylisonitril)Palladium(O) und Bis-(p-Methoxyphenylisonitril)Palladium(O). Davon sind Bis-(Dibenzylidenaceton)Palladium(O), Bis-(Cyclohexylisonitril)-Palladium(O) und Bis-(Isopropylisonitril)Palladium(O) bevorzugt.

Als Katalysatoren für die Umsetzung gemäß Verfahrensvariante b) können z. B. Palladiumkomplexe der in der US-Patentschrift 3 922 299 beschriebenen Art, vor allem Komplexe von $Pd(OOC-C_{1-12}$-Alkyl$)_2$, besonders Palladiumacetat, mit 3wertigen Phosphor- oder Arsenverbindungen, wie Trialkyl-, Triaryl-, Trialkoxy-, Triphenoxy-, Trihalogenphosphinen oder -arsinen oder gemischt substituierten dreiwertigen Phosphor- oder Arsenverbindungen, verwendet werden. Als Beispiele solcher Phosphor- oder Arsenverbindungen seien genannt: Triphenylarsin, Diphenylmethylphosphin, Diphenylmethoxyphosphin, Trimethylphosphin, Triäthylphosphin, Tri-n-butylphosphin, Triphenylphosphin, Phenyl-di-n-butoxyphosphin, Phosphortrichlorid, Phenyldichlorphosphin und Tri-o-tolylphosphin. Die genannten Komplexe können als solche eingesetzt oder in situ, d. h. im Reaktionsmedium, gebildet werden. Besonders bevorzugt verwendet man ein Gemisch aus Palladiumacetat und Triphenylphosphin oder Tri-o-tolylphosphin.

Bei der Herstellungsmethode a) verwendet man als Katalysatoren bevorzugt $PDCl_2$, $PdBr_2$, $Pd(OOCCH_3)_2$, $Pd(CH_3COCHCOCH_3)_2$, $Pd(OOCCH_3)_2$(2,2'-Bipyridyl), $PdCl_2(NC-\text{Phenyl})_2$, Bis-(Dibenzylidenaceton)Palladium(O) und Bis-(Cyclohexylisonitril)Palladium(O). Ganz besonders bevorzugt sind $PdCl_2$, Palladiumacetat und Bis-(Dibenzylidenaceton)Palladium(O). Bevorzugte Katalysatoren für die Herstellungsmethode b) sind Gemische aus Palladiumacetat und Triphenylphosphin oder Tri-o-tolylphosphin.

Die Katalysatoren werden im allgemeinen in einer Menge von 0,0001 bis 20 Mol%, bevorzugt etwa 0,001 bis 3 Mol%, bezogen auf die Verbindung der Formel IIa bzw. IIb, eingesetzt.

Als Basen können im erfindungsgemäßen Verfahren sowohl anorganische als auch organische Verbindungen, die im Reaktionsmedium ausreichend löslich sind, verwendet werden. Geeignete Basen sind beispielsweise Verbindungen der Formeln V bis VII

$$(Z')^{n\oplus}({}^{\ominus}OOCQ')_{m_1} \qquad (V)$$

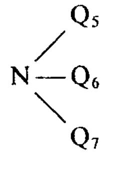

(VI)

oder

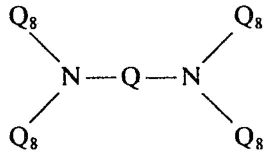

(VII)

sowie cyclische tertiäre Amine, z. B. N-Methyl- oder N-Äthylpiperidin, 1,2,2,6,6-Pentamethylpiperidin, 4-Oxo-1,2,2,6,6-pentamethylpiperidin, 1,4-Diazabicyclo[2,2,2]octan (DABCO), N-Alkylmorpholine und N-Alkylpyrrolidine, wie N-Methyl- und N-Äthylmorpholin, N-Methyl- und N-Äthylpyrrolidin, oder N,N-Dialkylpiperazine, wie N,N'-Dimethylpiperazin.

In den obigen Formeln bedeuten:

$m_1$ die Zahl 1 oder 2,
Q' Phenyl oder $C_{1-17}$-Alkyl,
Z' ein Alkalimetallkation, ein Erdalkalimetallkation oder

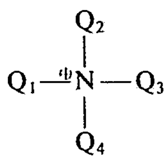

Q geradkettiges oder verzweigtes Alkylen mit 2—6 C-Atomen,
$Q_1$ Wasserstoff, $C_{1-12}$-Alkyl, Cyclopentyl, Cyclohexyl, Benzyl oder Phenyl,
$Q_2$, $Q_3$ und $Q_4$ gleiches oder verschiedenes $C_{1-12}$-Alkyl,
$Q_5$ $C_{1-12}$-Alkyl, Cyclopentyl, Cyclohexyl, Phenyl oder Benzyl, das auch substituiert sein kann, beispielsweise durch ein Halogenatom, wie Chlor oder Brom, eine Alkyl- oder Alkoxygruppe mit je 1—4 und besonders 1 oder 2 C-Atomen,
$Q_6$ und $Q_7$ gleiches oder verschiedenes $C_{1-12}$-Alkyl und
$Q_8$ Methyl oder Äthyl.

Z' in der Bedeutung eines Alkalimetallkations ist besonders das Natrium- und vor allem das Lithiumkation. Alkylgruppen Q' und $Q_1$ bis $Q_7$ können geradkettig oder verzweigt sein. Stellen $Q_5$ bis $Q_7$ Alkylgruppen dar, so weisen diese mit Vorteil zusammen mindestens 9 C-Atome auf, während Alkylgruppen $Q_1$ bis $Q_4$ bevorzugt je 1—4 C-Atome aufweisen. Beispiele von Verbindungen der Formeln V bis VII sind: das Lithiumacetat, -butyrat und -stearat, das Barium- und Calciumacetat, das Kalium-, Calcium- und Natriumstearat, das Lithium- und Natriumbenzoat, sowie die entsprechenden Trimethyl-, Tetramethyl-, Tetraäthyl- und Tetra-n-butylammoniumsalze; Triäthylamin, Tri-n-butylamin, Tri-(2-äthylhexylamin), Tri-n-octylamin und Tri-n-dodecylamin; N-Benzyldialkylamine, wie N-Benzyldimethylamin, N-Benzyldiäthylamin, N-(4-Chlorbenzyl)-dimethylamin und N-(3-Methylbenzyl- oder 3-Methoxybenzyl)-dimethylamin; N,N,N',N'-Tetramethyl- und N,N,N',N'-Tetraäthyläthylendiamin, N,N,N',N'-Tetramethyl-1,3-diaminopropan und N,N,N',N'-Tetramethyl-1,6-diaminohexan. Für die Herstellungsmethode b) kommen auch andere anorganische Basen, vor allem wasserlösliche, wie Alkalimetallcarbonate oder -hydrogencarbonate, in Betracht.

Bevorzugt verwendet man als Basen tertiäre Amine der vorerwähnten Art, vor allem N-Äthylmorpholin oder Verbindungen der Formel VI, worin $Q_5$ 4-Chlorbenzyl, 4-Methylbenzyl oder 4-Methoxybenzyl und insbesondere Benzyl und $Q_6$ und $Q_7$ je Alkyl mit 1—4 C-Atomen, vor allem 1 oder 2 C-Atomen, darstellen, oder worin $Q_5$, $Q_6$ und $Q_7$ je Alkyl mit 3—12 C-Atomen darstellen. Besonders bevorzugt sind N-Benzyldimethylamin, N-Äthylmorpholin und Tri-n-butylamin.

Die Reaktionstemperaturen für die erfindungsgemäße Umsetzung liegen zweckmäßig zwischen 0 und 200°C, vorzugsweise zwischen 90 und 150°C.

Sind die Verbindungen der Formel IIa bzw. IIb flüssig, so kann die Umsetzung ohne Zusatz eines Lösungsmittels durchgeführt werden. Bevorzugt wird die Reaktion jedoch in einem gegenüber den Reaktionspartnern inerten organischen Lösungsmittel durchgeführt. Geeignete inerte organische Lösungsmittel sind z. B. gegebenenfalls chlorierte aliphatische, cycloaliphatische oder aromatische Kohlenwasserstoffe, wie n-Pentan, n-Heptan, n-Octan, Cyclopentan, Cyclohexan, Benzol, Toluol, Xylole und Chlorbenzol; aromatische, aliphatische und cyclische Äther, wie Anisol, Diäthyläther, Di-isopropyläther, Tetrahydrofuran und Dioxan; Nitrile, besonders Benzonitril und Alkylnitrile mit 2 bis 5 C-Atomen, wie Acetonitril, Propionitril und Butyronitril; 3-Methoxypropionitril und 3-Äthoxypropionitril; N,N-Dialkylamide von aliphatischen Monocarbonsäuren mit 1 bis 3 C-Atomen im Säureteil, wie N,N-Dimethylformamid und N,N-Dimethylacetamid; Alkohole mit bis zu 8 C-Atomen, wie Äthanol, Propanol und tert.-Butanol; aliphatische und cycloaliphatische Ketone, wie Aceton, Diäthylketon, Methylisopropylketon, Cyclopentanon und Cyclohexanon; Ester, wie Ester der Kohlensäure, z. B. Diäthylcarbonat, und

Alkyl- oder Alkoxyalkylester von aliphatischen Monocarbonsäuren mit insgesamt 2 bis 8 C-Atomen, wie Essigsäuremethyl-, -äthyl-, -n-butyl- und -isobutylester, Buttersäureäthyl- und -n-butylester, sowie 1-Acetoxy-2-methoxyäthan. Bevorzugte Lösungsmittel sind je nach Herstellungsmethode Nitrile, Ketone, Ester, cyclische Äther und aromatische Kohlenwasserstoffe der oben erwähnten Art. Für die Umsetzung in Gegenwart anorganischer Basen eignen sich vor allem polare Lösungsmittel, wie Nitrile, Ketone und Ester. Ganz besonders bevorzugt wird die Umsetzung in Gegenwart einer organischen Base und eines aromatischen Äthers oder Kohlenwasserstoffs, vor allem Anisol, Xylolen oder Toluol vorgenommen. Es kann auch in einem Zweiphasensystem gearbeitet werden, z. B. in einem Gemisch aus Wasser und einem mit Wasser nicht mischbaren Lösungsmittel, wie Wasser mit Chlorbenzol, Xylolen oder Toluol.

Die Verbindungen der Formel I sind – wie schon erwähnt – wertvolle Zwischenprodukte zur Herstellung von optischen Aufhellern, besonders asymmetrischen Divinylstilbenaufhellern. Derartige Aufheller können z. B. dadurch erhalten werden, daß man eine Verbindung der Formel I unter den gleichen Reaktionsbedingungen wie im Vorangehenden für die Verfahrensvariante b) beschrieben mit einer Verbindung der Formel IIIb umsetzt, wobei diese Verbindung der Formel IIIb auch von der für die Umsetzung mit Verbindungen der Formel IIb eingesetzten verschieden sein kann.

## Beispiel 1

Zu 100 ml p-Xylol werden unter Argon 14,83 g (50 mMol) des Säurechlorids der Formel

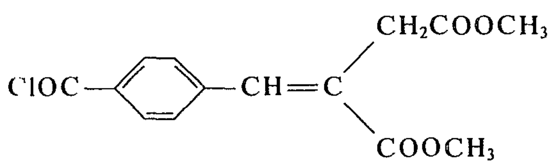

(100)

10,17 g (50 mMol) 4-Bromstyrol, 6,76 g (50 mMol) N-Benzyldimethylamin und 0,1122 g (0,5 mMol) Palladiumacetat gegeben. Das Reaktionsgemisch wird während 2 Std. bei 130°C gerührt, dann zweimal mit je 50 ml 2N HCl und zweimal mit je 50 ml 2N NaOH ausgeschüttelt, über Magnesiumsulfat getrocknet und eingedampft. Nach dem Umkristallisieren aus Cyclohexan erhält man 10,5 g (51% d. Th.) der Verbindung der Formel

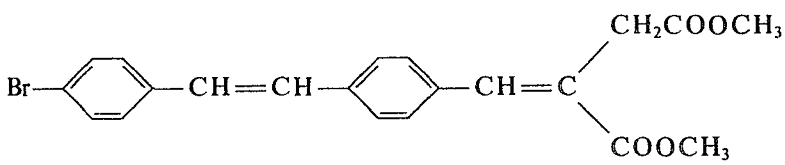

(101)

als hellgelbe Kristalle; Fp. 133,3°C.

Das Säurechlorid der Formel (100) und die entsprechende freie Säure sind neu und können wie folgt hergestellt werden:

25,15 g (125 mMol) 4-Brombenzoesäure, 24,7 g (156,25 mMol) Itaconsäuredimethylester, 26,55 g (262,5 mMol) Triäthylamin, 0,280 g (1,25 mMol) Palladiumacetat und 1,52 g (5 mMol) Tri-o-tolylphosphin werden 3,5 Stunden bei 100°C gerührt. Das Reaktionsgemisch wird zu 150 ml 2N HCl gegeben, das Rohprodukt wird abfiltriert und aus Toluol umkristallisiert. Man erhält 25,8 g (74% d. Th.) der Verbindung der Formel

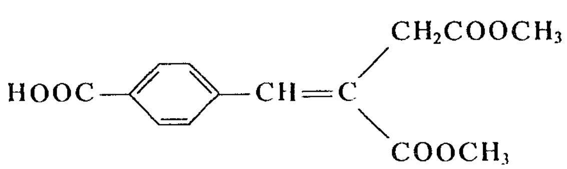

(102)

in Form weißer Kristalle vom Fp. 147,0°C.

30,2 g (109 mMol) der Verbindung (102), 16,17 g (136,25 mMol) Thionylchlorid, 200 ml Toluol und 0,25 ml N,N-Dimethylformamid werden während 3 Std. bei 20°C gerührt. Das Reaktionsgemisch wird eingedampft und aus Cyclohexan umkristallisiert. Man erhält 29,5 g (91% d. Th.) der Verbindung (100) als gelbe Kristalle vom Fp. 81,0°C.

## Beispiel 2

Zu 30 ml p-Xylol werden unter Argon 3,7 g (15 mMol) Stilben-4-carbonsäurechlorid, 3,05 g

(15 mMol) 4-Bromstyrol, 2,03 g (15 mMol) N-Benzyldimethylamin und 0,0336 g (0,15 mMol) Palladiumacetat gegeben. Das Reaktionsgemisch wird während 1 Std. bei 130°C gerührt. Nach der Aufarbeitung wie in Beispiel 1 beschrieben erhält man 3,2 g (59% d. Th.) der Verbindung der Formel

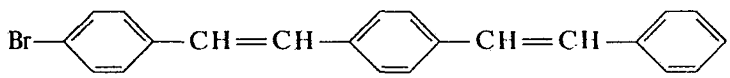 (200)

als grün-gelbe Kristalle; Fp. 283,7°C.

Das Stilben-4-carbonsäurechlorid kann wie folgt hergestellt werden:

25,15 g (125 mMol) 4-Brombenzoesäure, 16,25 g (156,25 mMol) Styrol, 26,55 g (262,5 mMol) Triäthylamin, 0,28 g (1,25 mMol) Palladiumacetat und 1,52 g (5 mMol) Tri-o-tolylphosphin werden 3,5 Std. bei 100°C gerührt. Das Reaktionsgemisch wird in 100 ml 2N HCl eingegossen, und das Rohprodukt wird abfiltriert und aus Tetrahydrofuran/Toluol umkristallisiert. Man erhält 15,3 g (55% d. Th.) Stilben-4-carbonsäure in Form weißer Kristalle; Fp. 252,4°C.

15,1 g (67,4 mMol) Stilben-4-carbonsäure, 10,0 g (84,25 mMol) Thionylchlorid, 150 ml Toluol und 0,18 ml N,N-Dimethylformamid werden 3 Std. bei 70°C gerührt. Danach wird das Reaktionsgemisch eingedampft, und das Rohprodukt wird aus n-Hexan/Cyclohexan umkristallisiert. Man erhält 13,7 g (84% d. Th.) Stilben-4-carbonsäurechlorid in Form weißer Kristalle; Fp. 131,2°C.

## Beispiel 3

4-Bromstilben-4'-acrylsäureäthylester. Zu 50 ml p-Xylol werden unter Argon 25,8 g (0,1 Mol) 4,4'-Dibromstilben, 10 g (0,1 Mol) Acrylsäureäthylester, 18,5 g (0,1 Mol) Tri-n-butylamin, 0,224 g (0,001 Mol) Palladiumacetat und 0,6 g (0,002 Mol) Tri-o-tolylphosphin gegeben. Das Reaktionsgemisch wird 4 Std. bei 90°C gerührt. Nach der Aufarbeitung erhält man 2 g (6% d. Th.) 4-Bromstilben-4'-acrylsäureäthylester. Fp. 166,9°C.

## Beispiel 4

4-Bromstilben-4'-acrylnitril. Zu 10 ml Toluol werden unter Argon 6,76 g (0,02 Mol) 4,4'-Dibromstilben, 1,06 g (0,02 Mol) Acrylnitril, 4,08 g (0,022 Mol) Tri-n-butylamin, 0,0898 g (0,4 mMol) Palladiumacetat und 0,2098 g (0,8 mMol) Triphenylphosphin gegeben. Das Reaktionsgemisch wird während 7 Std. bei 100–115°C gerührt. Nach dem Aufarbeiten erhält man 1 g (16% d. Th.) 4-Bromstilben-4'-acrylnitril in Form eines hellgelben Pulvers. Fp. 258°C.

## Beispiel 5

Zu 70 ml p-Xylol werden unter Argon 7,5 g (27 mMol) des Säurechlorids der Formel

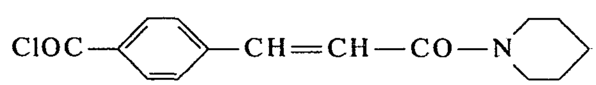 (500)

5,45 g (27 mMol) 4-Bromstyrol, 3,62 g (27 mMol) N-Benzyldimethylamin und 0,0604 g (0,27 mMol) Palladiumacetat gegeben. Das Reaktionsgemisch wird während 1,5 Stunden bei 130°C gerührt. Anschließend wird das ausgefallene Rohprodukt bei Raumtemperatur abfiltriert. Nach dem Umkristallisieren aus Tetrachlorkohlenstoff/n-Hexan erhält man 2,1 g (20% d. Th.) der Verbindung der Formel

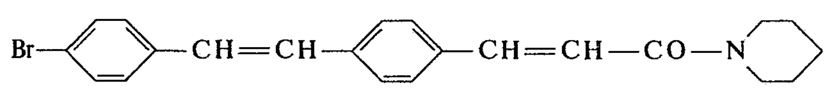 (501)

in Form heller grüngelber Kristalle; Fp. 214,9°C.

Das Säurechlorid der Formel (500) und die entsprechende freie Säure sind neu und können wie folgt hergestellt werden:

20,12 g (0,1 Mol) 4-Brombenzoesäure, 17,38 g (125 mMol) Acrylsäurepiperidid, 25,28 g (250 mMol) Triäthylamin, 0,224 g (1 mMol) Palladiumacetat und 1,216 g (4 mMol) Tri-o-tolylphosphin werden 15 Minuten bei 115°C gerührt. Das Gemisch wird mit 300 ml 2N Salzsäure gerührt, und das Rohprodukt wird abfiltriert. Es wird in 200 ml 2N Natronlauge gelöst und filtriert. Zum Filtrat werden 26 ml 10N Salzsäure zugegeben. Der Niederschlag wird abfiltriert, mit Wasser gewaschen und getrocknet. Man erhält 25,27 g (98% d. Th.) Zimtsäurepiperidid-4-carbonsäure als weiße Kristalle; Fp. 275,1°C.

21,43 g (82,7 mMol) der obigen Verbindung, 7,52 ml (103,4 mMol) Thionylchlorid, 120 ml Toluol und 0,3 ml N,N Dimethylformamid werden während 2 Std. bei 80–100°C gerührt. Das Reaktions

gemisch wird eingedampft und aus n-Hexan umkristallisiert. Man erhält 7,51 g (33% d. Th.) der Verbindung der Formel (500) in Form gelber Kristalle; Fp. 105°C.

Beispiel 6

Zu 50 ml p-Xylol werden unter Argon 4,77 g (20 mMol) Zimtsäureäthylester-4-carbonsäurechlorid, 4,04 g (20 mMol) 4-Bromstyrol, 2,72 g (20 mMol) N-Benzyldimethylamin und 0,0448 g (0,2 mMol) Palladiumacetat zugegeben, und das Reaktionsgemisch wird während 1,5 Std. bei 130°C gerührt. Bei Raumtemperatur wird das ausgefallene Rohprodukt abfiltriert und anschließend aus Methanol umkristallisiert. Man erhält 0,73 g (10% d. Th.) 4-Bromstilben-4'-acrylsäureäthylester in Form gelber Kristalle; Fp. 166,9°C.

Das Zimtsäureäthylester-4-carbonsäurechlorid kann wie folgt hergestellt werden:

10,05 g (50 mMol) 4-Brombenzoesäure, 6,25 g (62,5 mMol) Acrylsäureäthylester, 12,6 g (125 mMol) Triäthylamin, 0,1122 g (0,5 mMol) Palladiumacetat und 0,608 g (2 mMol) Tri-o-tolylphosphin in 20 ml Toluol werden während 1,5 Std. unter Rückfluß gekocht. Das Reaktionsgemisch wird mit 200 ml 2N Salzsäure ausgeschüttelt, und das Rohprodukt wird abfiltriert und aus Toluol umkristallisiert. Man erhält 10,1 g (92% d. Th.) Zimtsäureäthylester-4-carbonsäure als weiße Kristalle; Fp. 196–197°C.

14,8 g (67 mMol) Zimtsäureäthylester-4-carbonsäure, 9,57 g (80 mMol) Thionylchlorid, 100 ml Toluol und 0,18 ml N,N-Dimethylformamid werden während 30 Minuten unter Rückfluß gekocht. Das Reaktionsgemisch wird eingedampft, und das Rohprodukt wird aus n-Hexan umkristallisiert. Man erhält 12,6 g (79% d. Th.) Zimtsäureäthylester-4-carbonsäurechlorid als weiße Kristalle; Fp. 76,8°C.

Beispiel 7

Zu 30 ml p-Xylol werden unter Argon 2,13 g (11,1 mMol) Zimtsäurenitril-4-carbonsäurechlorid, 2,24 g (11,1 mMol) 4-Bromstyrol, 1,51 g (11,1 mMol) N-Benzyldimethylamin und 0,0246 g (0,11 mMol) Palladiumacetat zugegeben, und das Reaktionsgemisch wird während 1,5 Std. bei 130°C gerührt. Das ausgefallene Rohprodukt wird bei Raumtemperatur abfiltriert und aus Methanol und dann aus Tetrachlorkohlenstoff umkristallisiert. Man erhält 0,38 g (11% d. Th.) 4-Bromstilben-4'-acrylsäurenitril; Fp. 258°C.

Das Zimtsäurenitril-4-carbonsäurechlorid kann wie folgt hergestellt werden:

10,05 g (50 mMol) 4-Brombenzoesäure, 3,31 g (62,5 mMol) Acrylnitril, 12,6 g (125 mMol) Triäthylamin, 0,112 g (0,5 mMol) Palladiumacetat, 0,608 g (2 mMol) Tri-o-tolylphosphin und 20 ml Toluol werden während 6 Std. unter Rückfluß gekocht. Das Reaktionsgemisch wird mit 100 ml 2N Salzsäure ausgeschüttelt, das Rohprodukt wird abfiltriert und aus Methanol umkristallisiert. Man erhält 3 g (35% d. Th.) Zimtsäurenitril-4-carbonsäure als hellgelbe Kristalle; Fp. 281°C.

14,3 g (83 mMol) Zimtsäurenitril-4-carbonsäure, 11,9 g (100 mMol) Thionylchlorid, 100 ml Toluol und 0,18 ml N,N-Dimethylformamid werden während 15 Minuten unter Rückfluß gekocht und anschließend eingedampft. Nach zweimaligem Umkristallisieren aus Tetrachlorkohlenstoff erhält man 7,7 g (48% d. Th.) Zimtsäurenitril-4-carbonsäurechlorid als gelbe Kristalle; Fp. 161,0°C.

Beispiel 8

Zu 90 ml p-Xylol werden unter Argon 8,54 g (35 mMol) des Säurechlorids der Formel

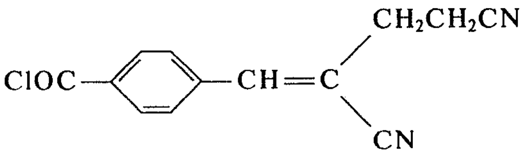

(800)

7,06 g (35 mMol) 4-Bromstyrol, 4,76 g N-Benzyldimethylamin und 0,0784 g (0,35 mMol) Palladiumacetat gegeben. Das Reaktionsgemisch wird während 4,5 Std. bei 130°C gerührt. Das Rohprodukt wird bei Raumtemperatur abfiltriert und mit 50 ml 2N Salzsäure gewaschen. Anschließend wird es aus Äthylmethylketon umkristallisiert. Man erhält 3,2 g (25% d. Th.) der Verbindung der Formel

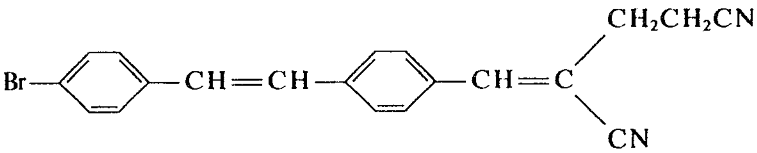

(801)

in Form gelbgrüner Kristalle; Fp. 177,2°C.

Das Säurechlorid der Formel (800) und die entsprechende freie Säure sind neu und können wie folgt hergestellt werden:

20,12 g (0,1 Mol) 4-Brombenzoesäure, 13,25 g (0,125 Mol) 2-Methylenglutarsäuredinitril, 21,23 g (0,21 Mol) Triäthylamin, 0,224 g (1 mMol) Palladiumacetat, 1,216 g (4 mMol) Tri-o-tolylphosphin und 100 ml p-Xylol werden während 18 Std. bei 115°C gerührt. Das erhaltene Zwischenprodukt (freie Säure) wird abfiltriert und mit 100 ml 2N Salzsäure gewaschen. Es wird dann in 160 ml 2N Natronlauge gelöst und durch Zugabe von 40 ml 10N Salzsäure wieder ausgefällt. Nach dem Abfiltrieren und Trocknen werden 10,26 ml (0,14 Mol) Thionylchlorid, 150 ml Toluol und 0,3 ml N,N-Dimethylformamid zugegeben, und das Gemisch wird während 2 Std. bei 90°C gerührt. Das Reaktionsgemisch wird eingedampft, und das Rohprodukt wird aus einem Gemisch von 125 ml Tetrachlorkohlenstoff und 35 ml Toluol umkristallisiert. Man erhält 6,24 g (26% d. Th.) der Verbindung der Formel (800) als hellbraune Kristalle; Fp. 64,6°C.

**Patentansprüche**

1. Verbindungen der Formel I

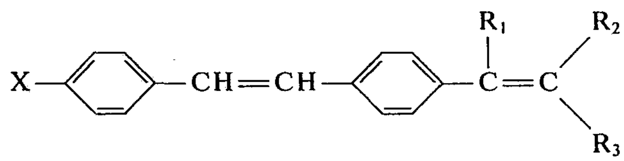

(I)

worin

X Brom oder Jod,
$R_1$ Wasserstoff, $C_{1-6}$-Alkyl oder eine nicht chromophore veresterte Carboxylgruppe,
$R_2$ unsubstituiertes oder nicht chromophor substituiertes $C_{1-6}$-Alkyl, nicht chromophor substituiertes $C_{2-4}$-Alkenyl, Phenyl oder durch Fluor, Chlor, $C_{1-4}$-Alkoxy, $C_{2-6}$-Alkoxyalkoxy, Di-($C_{1-4}$-alkyl)-amino oder $C_{1-4}$-Alkoxycarbonyl substituiertes Phenyl, oder einen nicht chromophoren Substituenten zweiter Ordnung und
$R_3$ Wasserstoff oder unsubstituiertes oder nicht chromophor substituiertes $C_{1-6}$-Alkyl oder nicht chromophor substituiertes $C_{2-4}$-Alkenyl bedeuten.

2. Verbindungen der Formel I nach Anspruch 1, worin X Brom bedeutet.

3. Verbindungen der Formel I nach Anspruch 1, worin X Brom, $R_1$ Wasserstoff, $C_{1-6}$-Alkyl oder $-COOR'''$, $R_2$ unsubstituiertes oder durch $C_{1-4}$-Alkoxy oder $C_{2-5}$-Alkoxycarbonyl substituiertes $C_{1-6}$-Alkyl, $-CN$, $-CF_3$, Phenyl oder eine Gruppe

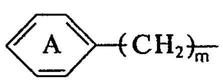

$-CO-R$, $-CO-NR'R''$ oder $-COOR'''$, $R_3$ Wasserstoff oder unsubstituiertes oder durch $C_{1-4}$-Alkoxy oder $C_{2-5}$-Alkoxycarbonyl substituiertes $C_{1-6}$-Alkyl, R Methyl, unsubstituiertes oder durch $C_{1-4}$-Alkoxy, $C_{4-6}$-Alkoxyalkoxy substituiertes $C_{2-6}$-Alkyl, einen Rest der Formel

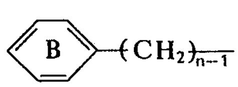

oder Naphthyl, R' Wasserstoff, Methyl, unsubstituiertes oder durch $C_{1-4}$-Alkoxy, $C_{4-6}$-Alkoxyalkoxy oder Di-($C_{1-4}$-Alkyl)-amino substituiertes $C_{2-6}$-Alkyl, unsubstituiertes oder methyl- oder äthylsubstituiertes Cyclohexyl, einen Rest der Formel

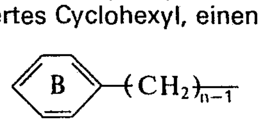

oder zusammen mit R'' $-(CH_2)_4-$ oder $-(CH_2)_5-$, R'' Wasserstoff, Methyl, unsubstituiertes oder durch $C_{1-4}$-Alkoxy oder $C_{4-6}$-Alkoxyalkoxy substituiertes $C_{2-6}$-Alkyl oder zusammen mit R' $-(CH_2)_4-$ oder $-(CH_2)_5-$, R''' Methyl, unsubstituiertes oder durch $C_{1-4}$-Alkoxy oder $C_{3-6}$-Alkoxyalkoxy substituiertes $C_{2-6}$-Alkyl, unsubstituiertes oder methyl- und/oder äthylsubstituiertes Cyclohexyl oder einen Rest der Formel

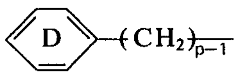

m die Zahl 1 oder 2 und n und p unabhängig voneinander die Zahl 1, 2 oder 3 bedeuten, wobei die Ringe A, B und D durch $C_{1-3}$-Alkyl und/oder Fluor oder Chlor mono- oder disubstituiert und die Ringe B und D, wenn n und p je die Zahl 1 bedeuten, durch $C_{1-3}$-Alkyl und/oder Fluor oder Chlor trisubstituiert oder durch $C_{1-3}$-Alkoxy mono- oder disubstituiert sein können.

4. Verbindungen der Formel I nach Anspruch 1, worin X Brom, $R_1$ Wasserstoff oder $C_{1-4}$-Alkyl, $R_2$, —CN, Phenyl, —CONR'R'', —COOR''' oder —CO—R und $R_3$ Wasserstoff, $C_{1-4}$-Alkyl oder $-CH_2COO-C_{1-4}$-Alkyl bedeuten, R Methyl, unsubstituiertes oder durch $C_{1-4}$-Alkoxy oder $C_{4-6}$-Alkoxyalkoxy substituiertes $C_{2-6}$-Alkyl, Naphthyl oder unsubstituiertes oder durch Fluor, Chlor, $C_{1-3}$-Alkyl und/oder $C_{1-3}$-Alkoxy substituiertes Phenyl, R' und R'' je Methyl oder Äthyl oder zusammen $-(CH_2)_4-$ oder $-(CH_2)_5-$, R''' Methyl oder unsubstituiertes oder durch $C_{1-4}$-Alkoxy oder $C_{3-6}$-Alkoxyalkoxy substituiertes $C_{2-6}$-Alkyl, m die Zahl 1, n und p unabhängig voneinander die Zahl 2 und insbesondere die Zahl 1 darstellen.

5. Verbindungen der Formel I nach Anspruch 1, worin X Brom, $R_1$ Wasserstoff oder $C_{1-4}$-Alkyl, $R_2$, —CN, Phenyl, —CONR'R'', —COOR''' oder —CO—R und $R_3$ Wasserstoff, $C_{1-4}$-Alkyl oder $-CH_2COO-C_{1-4}$-Alkyl bedeuten, wobei mindestens eines von $R_1$ und $R_3$ Wasserstoff ist, R $C_{1-6}$-Alkyl oder unsubstituiertes oder durch Fluor, Chlor, $C_{1-3}$-Alkyl oder $C_{1-3}$-Alkoxy substituiertes Phenyl, insbesondere unsubstituiertes $C_{1-4}$-Alkyl oder unsubstituiertes oder durch Chlor, Methyl oder Methoxy monosubstituiertes Phenyl, R' und R'' je Methyl oder Äthyl oder zusammen $-(CH_2)_5-$ und R''' Methyl oder unsubstituiertes oder durch $C_{1-4}$-Alkoxy oder $C_{3-6}$-Alkoxyalkoxy substituiertes $C_{2-6}$-Alkyl, insbesondere unsubstituiertes $C_{1-4}$-Alkyl bedeuten.

6. Verbindungen der Formel I nach Anspruch 1, worin X Brom, $R_1$ Methyl und insbesondere Wasserstoff, $R_2$ Phenyl, —CN,

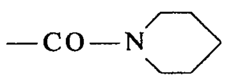

oder —COOR''', R''' unsubstituiertes $C_{1-4}$-Alkyl und $R_3$ Wasserstoff, Methyl, $-CH_2COOCH_3$ oder $-CH_2COOC_2H_5$ bedeuten, wobei mindestens eines von $R_1$ und $R_3$ Wasserstoff ist.

7. Die Verbindung nach Anspruch 1, der Formel

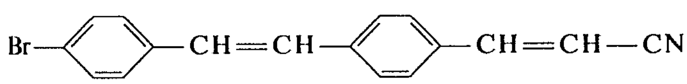

8. Die Verbindung nach Anspruch 1, der Formel

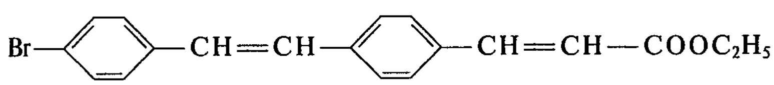

9. Verfahren zur Herstellung von Verbindungen der Formel I nach Anspruch 1, dadurch gekennzeichnet, daß man entweder

a) eine Verbindung der Formel IIa

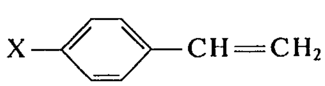

(IIa)

in Gegenwart einer Base und unter Zusatz von Palladium-Metall oder von Palladiumverbindungen, die unter den Reaktionsbedingungen phosphorfreie labile Palladium(O)Verbindungen bilden, als Katalysator, mit einer Verbindung der Formel IIIa

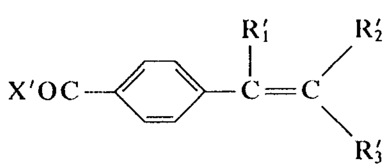

(IIIa)

oder

b) eine Verbindung der Formel IIb

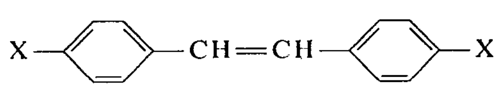

(IIb)

in Gegenwart einer Base und einer arsen- oder phosphorhaltigen Palladiumverbindung als Katalysator mit einer Verbindung der Formel IIIb

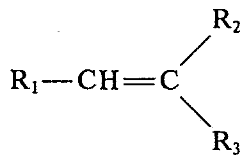

(IIIb)

umsetzt, wobei X, $R_1$, $R_2$ und $R_3$ die im Anspruch 1 angegebene Bedeutung haben, X' Chlor, Brom oder Jod darstellt und $R_1'$, $R_2'$ und $R_3'$ dieselbe Bedeutung wie $R_1$, $R_2$ und $R_3$ haben können, jedoch frei von Substituenten sind, welche zur Reaktion mit Gruppen X'OC— befähigt sind.

10. Verfahren nach Anspruch 9, dadurch gekennzeichnet, daß man eine Verbindung der Formel IIIa verwendet, worin X' Chlor bedeutet.

11. Verfahren nach Anspruch 10, dadurch gekennzeichnet, daß man 4-Bromstyrol als Verbindung IIa verwendet.

12. Verfahren nach Anspruch 9, dadurch gekennzeichnet, daß man bei der Verfahrensvariante a) als Palladiumverbindung $PDCl_2$, $PdBr_2$, $PD(OOCCH_3)_2$, $Pd(CH_3COCHCOCH_3)_2$, $Pd(OOCCH_3)_2$(2,2'-Bipyridyl), $PdCl_2$(NC—Phenyl)$_2$, Bis-(Dibenzylidenaceton)Palladium(O) oder Bis-(Cyclohexylisonitril)-Palladium(O) verwendet.

13. Verfahren nach Anspruch 9, dadurch gekennzeichnet, daß man bei der Verfahrensvariante a) als Palladiumverbindung $PdCl_2$, Palladiumacetat oder Bis-(Dibenzylidenaceton)Palladium(O) verwendet.

14. Verfahren nach Anspruch 9, dadurch gekennzeichnet, daß man bei der Verfahrensvariante b) als Katalysator ein Gemisch von Palladiumacetat und Triphenylphosphin oder Tri-o-tolylphosphin verwendet.

15. Verfahren nach Anspruch 9, dadurch gekennzeichnet, daß man als Base N-Benzyldimethylamin, N-Äthylmorpholin oder Tri-n-butylamin verwendet.

16. Verfahren nach Anspruch 9, dadurch gekennzeichnet, daß man den Katalysator in einer Menge von 0,001 bis 3 Mol%, bezogen auf die Verbindung der Formel IIa bzw. IIb, verwendet.

17. Die Verbindungen der Formeln

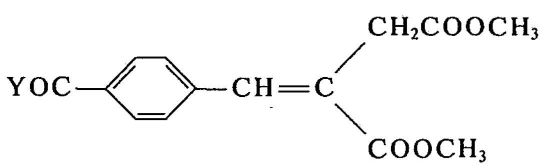

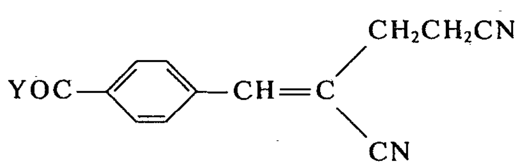

und

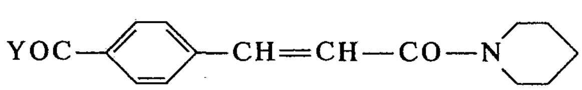

worin Y —OH oder Cl bedeutet.

## Claims

1. A compound of the formula I

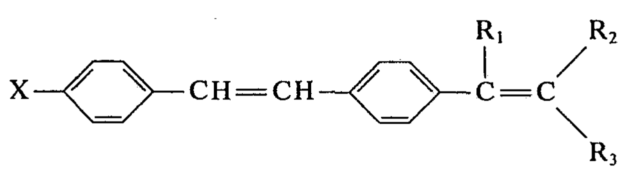

(I)

in which

X is bromine or iodine,

$R_1$ is hydrogen or $C_{1-6}$alkyl or a non-chromophoric esterified carboxyl group,

$R_2$ is $C_{1-6}$alkyl which is unsubstituted or substituted by non-chromophoric groups, or is $C_{2-4}$alkenyl which is substituted by non-chromophoric groups, or is phenyl or phenyl which is substituted by fluorine, chlorine, $C_{1-4}$alkoxy, $C_{2-6}$alkoxyalkoxy, di($C_{1-4}$alkyl)amino or $C_{1-4}$alkoxycarbonyl, or is a non-chromophoric substituent of the second order, and

$R_3$ is hydrogen or $C_{1-6}$alkyl which is unsubstituted or substituted by non-chromophoric groups or is $C_{2-4}$alkenyl which is substituted by non-chromophoric groups.

2. compound of the formula I according to claim 1, in which X is bromine.

3. A compound of the formula I according to claim 1, in which X is bromine, $R_1$ is hydrogen, $C_{1-6}$alkyl or $-COOR'''$, $R_2$ is $C_{1-6}$alkyl which is unsubstituted or substituted by $C_{1-4}$alkoxy or $C_{2-5}$alkoxycarbonyl, or is $-CN$, $-CF_3$, phenyl or a group

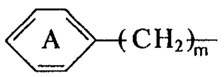

$-CO-R$, $-CO-NR'R''$ or $-COOR'''$, $R_3$ is hydrogen or $C_{1-6}$alkyl which is unsubstituted or substituted by $C_{1-4}$alkoxy or $C_{2-5}$alkoxycarbonyl, R is methyl, $C_{2-6}$alkyl which is unsubstituted or substituted by $C_{1-4}$alkoxy or $C_{4-6}$alkoxyalkoxy, or is a radical of the formula

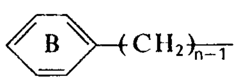

or naphthyl, R' is hydrogen, methyl, $C_{2-6}$alkyl which is unsubstituted or substituted by $C_{1-4}$alkoxy, $C_{4-6}$alkoxyalkoxy or di($C_{1-4}$alkyl)amino, or is cyclohexyl which is unsubstituted or substituted by methyl or ethyl, or is radical of the formula

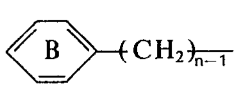

or, together with R'', is $-(CH_2)_4-$ or $-(CH_2)_5-$, R'' is hydrogen, methyl or $C_{2-6}$alkyl which is unsubstituted or substituted by $C_{1-4}$alkoxy or $C_{4-6}$alkoxyalkoxy or, together with R', is $-(CH_2)_4-$ or $-(CH_2)_5-$, R''' is methyl, $C_{2-6}$alkyl which is unsubstituted or substituted by $C_{1-4}$alkoxy or $C_{3-6}$alkoxyalkoxy, or is cycloalkyl which is unsubstituted or substituted by methyl and/or ethyl, or is radical of the formula

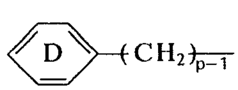

m is 1 or 2 and each of n and p independently is 1, 2 or 3, and the rings A, B and D may be monosubstituted or disubstituted by $C_{1-3}$alkyl and/or fluorine or chlorine, and, if n and p are each 1, the rings B and D may be trisubstituted by $C_{1-3}$alkyl and/or fluorine or chlorine or be monosubstituted or disubstituted by $C_{1-3}$alkoxy.

4. A compound of the formula I according to claim 1, in which X is bromine, $R_1$ is hydrogen or $C_{1-4}$alkyl, $R_2$ is $-CN$, phenyl, $-CONR'R''$, $-COOR'''$ or $-CO-R$ and $R_3$ is hydrogen, $C_{1-4}$alkyl or $-CH_2COO-C_{1-4}$alkyl, R is methyl, $C_{2-6}$alkyl which is unsubstituted or substituted by $C_{1-4}$alkoxy or $C_{4-6}$alkoxyalkoxy, or is naphthyl or phenyl which is unsubstituted or substituted by fluorine, chlorine $C_{1-3}$alkyl and/or $C_{1-3}$alkoxy R' and R'' are each methyl or ethyl or together are $(CH_2)_4-$ or $-(CH_2)_5-$, R''' is methyl or $C_{2-6}$alkyl which is unsubstituted or substituted by $C_{1-4}$alkoxy or $C_{3-6}$alkoxyalkoxy, m is 1, and each of n and p independently is 2, preferably 1.

5. A compound of the formula I according to claim 1, in which X is bromine, $R_1$ is hydrogen or $C_{1-4}$alkyl, $R_2$ is $-CN$, phenyl, $-CONR'R''$, $-COOR'''$ or $-CO-R$ and $R_3$ is hydrogen, $C_{1-4}$alkyl or $-CH_2COO-C_{1-4}$alkyl at least one of $R_1$ and $R_3$ being hydrogen, R is $C_{1-6}$alkyl or phenyl which is unsubstituted or substituted by fluorine, chlorine, $C_{1-3}$alkyl or $C_{1-3}$alkoxy, preferably unsubstituted $C_{1-4}$alkyl or phenyl which is unsubstituted or monosubstituted by chlorine, methyl or methoxy, R' and R'' are each methyl or ethyl or together are $-(CH_2)_5-$ and R''' is methyl or $C_{2-6}$alkyl which is unsubstituted or substituted by $C_{1-4}$alkoxy or $C_{3-6}$alkoxyalkoxy, preferably unsubstituted $C_{1-4}$alkyl.

6. A compound of the formula I according to claim 1, in which X is bromine, $R_1$ is methyl or, preferably, hydrogen, $R_2$ is phenyl, $-CN$,

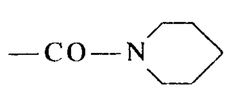

or $-COOR'''$, R''' is unsubstituted $C_{1-4}$alkyl, $R_3$ is hydrogen, methyl, $-CH_2COOCH_3$ or $-CH_2COOC_2H_5$, at least one of $R_1$ and $R_3$ being hydrogen.

7. The compound according to claim 1 of the formula

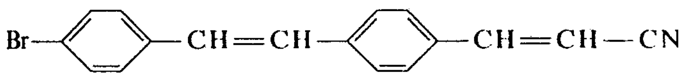

8. The compound according to claim 1 of the formula

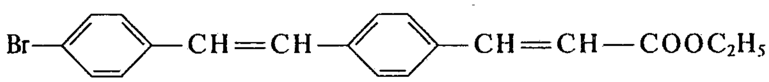

9. A process for the preparation of a compound of the formula I according to claim 1, which comprises either

a) reacting a compound of the formula IIa

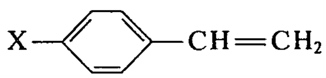

(IIa)

with a compound of the formula IIIa

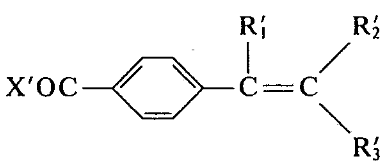

(IIIa)

in the presence of a base and with the addition, as catalyst, of a palladium metal or a palladium compound which forms a phosphorus-free labile palladium(O) compound under the reaction conditions, or

b) reacting a compound of the formula IIb

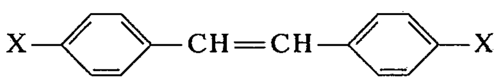

(IIb)

with a compound of the formula IIIb

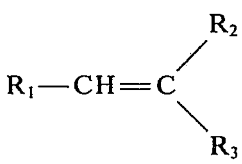

(IIIb)

in the presence of a base and, as catalyst, a palladium compound containing arsenic or phosphorus, in which formulae IIa, IIIa, IIb and IIIb, X, $R_1$, $R_2$ and $R_3$ are as defined in claim 1, X' is chlorine, bromine or iodine and $R'_1$, $R'_2$ and $R'_3$ can have the same meaning as $R_1$, $R_2$ and $R_3$, but are free of substituents capable of reacting with groups X'OC–.

10. A process according to claim 9, which comprises the use of a compound of the formula IIIa in which X' is chlorine.

11. A process according to claim 10, wherein the compound IIa is 4-bromostyrene.

12. A process according to claim 9, wherein the palladium compound in process variant a) is $PdCl_2$, $PdBr_2$, $Pd(OOCCH_3)_2$, $Pd(CH_3COCHCOCH_3)_2$, $Pd(OOCCH_3)_2$(2,2'-bipyridyl), $PdCl_2$(NC-phenyl)$_2$, bis(dibenzylideneacetone)palladium(O) or bis(cyclohexylisonitrile)palladium(O).

13. A process according to claim 9, wherein the palladium compound in the process variant a) is $PdCl_2$, palladium acetat or bis(dibenzylideneacetone)palladium(O).

14. A process according to claim 9, wherein the catalyst in the process variant b) is a mixture of palladium acetate and triphenylphosphine or tri-o-tolylphosphine.

15. A process according to claim 9, wherein the base is N-benzyldimethylamine, N-ethylmorpholine or tri-n-butylamine.

16. A process according to claim 9, wherein the catalyst is used in an amount of 0,001 to 3 mol%, based on the compound of the formula IIa or IIb.

17. The compounds of the formulae

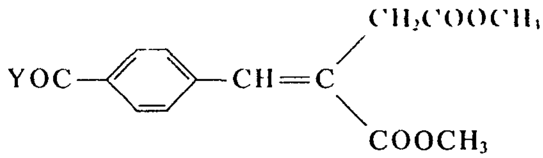

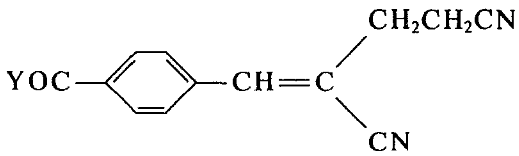

and

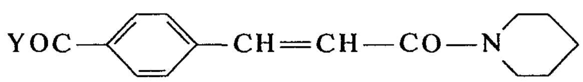

in which Y is $-OH$ or Cl.

## Revendications

1. Composés de formule I

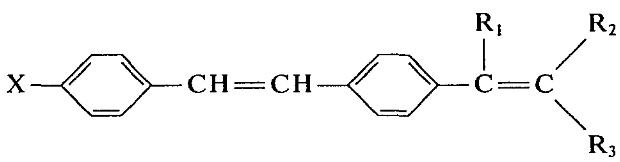

(I)

dans laquelle X est un atome de brome ou d'iode,

$R_1$ est un atome d'hydrogène, un groupe alkyl en $C_{1-6}$ ou bien un groupe carboxyle estérifié non chromophore,
$R_2$ est un groupe alkyle en $C_{1-6}$, non substitué ou substitué par un radical non chromophore, un groupe alcényle en $C_{2-4}$ substitué par un radical non chromophore, un groupe phényle ou bien un groupe phényle substitué par un atome de fluor, de chlore, un radical alcoxy en $C_{1-4}$, alcoxyalcoxy en $C_{2-6}$, di-(alkyl en $C_{1-4}$)-amino ou alcoxy (en $C_{1-4}$)carbonyle, ou bien un substituant non chromophore du deuxième ordre, et
$R_3$ est un atome d'hydrogène ou bien un groupe alkyle en $C_{1-6}$ non substitué ou substitué par un radical non chromophore ou bien groupe alcényle en $C_{2-4}$ substitué par un radical non chromophore.

2. Composés de formule I selon la revendication 1, dans lesquels X est un atome de brome.
3. Composés de formule I selon la revendication 1, dans lesquels X est un atome de brome; $R_1$ est un atome d'hydrogène, un groupe alkyle en $C_{1-6}$ ou $-COOR'''$, $R_2$ est un groupe alkyle en $C_{1-6}$ non substitué ou substitué par un radical alcoxy en $C_{1-4}$ ou alcoxycarbonyle en $C_{2-5}$, un groupe $-CN$, $-CF_3$, phényle ou bien un groupe

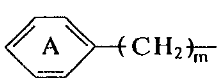

$-CO-R$, $-CO-NR'R''$ ou $-COOR'''$; $R_3$ est un atome d'hydrogène ou un groupe alkyle en $C_{1-6}$ non substitué ou substitué par un radical alcoxy en $C_{1-4}$ ou alcoxycarbonyle en $C_{2-5}$; R est le groupe méthyle, un groupe alkyle en $C_{2-6}$ non substitué ou substitué par un radical alcoxy en $C_{1-4}$, alcoxyalcoxy en $C_{4-6}$ un reste de formule

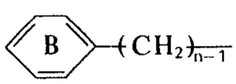

ou naphthyle; R' est un atome d'hydrogène, le groupe méthyle, un groupe alkyle en $C_{2-6}$ non substitué ou substitué par un radical alcoxy en $C_{1-4}$, alcoxyalcoxy en $C_{4-6}$, ou di-(alkyl en $C_{1-4}$)-amino, un groupe cyclohexyle non substitué ou méthylé ou éthylé, un reste de formule

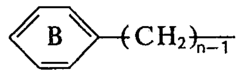

ou bien forme avec R″ un groupe $-(CH_2)_4-$ ou $-(CH_2)_5-$; R″ est un atome d'hydrogène, le groupe méthyle, un groupe alkyle en $C_{2-6}$ non substitué ou substitué par un radical alcoxy en $C_{1-4}$ ou alcoxyalcoxy en $C_{4-6}$, ou bien forme avec R′ un groupe $-(CH_2)_4-$ ou $-(CH_2)_5-$; R‴ est le groupe méthyle, un groupe alkyle en $C_{2-6}$ non substitué ou substitué par un radical alcoxy en $C_{1-4}$ ou alcoxyalcoxy en $C_{3-6}$, un groupe cyclohexyle non substitué ou bien méthylé et/ou éthylé, ou bien un reste de formule

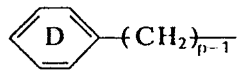

m est le nombre 1 ou 2, et n et p, indépendamment l'un de l'autre, sont les nombres 1, 2 ou 3, les noyaux A, B et D pouvant être mono- ou disubstitués par des radicaux alkyle en $C_{1-3}$ et/ou des atomes de fluor ou de chlore, et les noyaux B et D, quand n et p valent chacun 1, pouvant être trisubstitués par des radicaux alkyle en $C_{1-3}$ et/ou des atomes de fluor ou de chlore ou bien mono- ou disubstitués par des radicaux alcoxy en $C_{1-3}$.

4. Composés de formule I selon la revendication 1, dans lesquels X est un atome de brome; $R_1$ est un atome d'hydrogène ou un groupe alkyle en $C_{1-4}$; $R_2$ est un groupe $-CN$, phényle, $-CONR'R''$, $-COOR'''$ ou $-CO-R$, et $R_3$ est un atome d'hydrogène, un groupe alkyle en $C_{1-4}$ ou $-CH_2COO$ alkyle en $C_{1-4}$; R est le groupe méthyle, un groupe alkyle en $C_{2-6}$ non substitué ou substitué par des radicaux alcoxy en $C_{1-4}$ ou alcoxyalcoxy en $C_{4-6}$, un groupe naphthyle ou bien un groupe phényle non substitué ou substitué par des atomes de fluor, de chlore, par des groupes alkyle en $C_{1-3}$ et/ou alcoxy en $C_{1-3}$; R′ et R″ sont chacun le groupe méthyle ou éthyle ou bien forment ensemble les groupes $-(CH_2)_4-$ ou $-(CH_2)_5-$; R‴ est le groupe méthyle ou un groupe alkyle en $C_{2-6}$ non substitué ou substitué par des radicaux alcoxy en $C_{1-4}$ ou alcoxyalcoxy en $C_{3-6}$; m est le nombre 1; n et p, indépendamment l'un de l'autre, valent chacun 2 et en particulier valent 1.

5. Composés de formule I selon la revendication 1, dans lesquels X est un atome de brome; $R_1$ est un atome d'hydrogène ou un groupe alkyle en $C_{1-4}$; $R_2$ est un groupe $-CN$, phényle, $-CONR'R''$, $-COOR'''$ ou $-CO-R$, et $R_3$ est un atome d'hydrogène, un groupe alkyle en $C_{1-4}$ ou $-CH_2COO$ alkyle en $C_{1-4}$, au moins un des symboles $R_1$ et $R_3$ étant un atome d'hydrogène; R est un groupe alkyle en $C_{1-6}$ ou bien un groupe phényle non substitué ou substitué par des atomes de fluor, de chlore, des groupes alkyle en $C_{1-3}$ ou alcoxy en $C_{1-3}$, en particulier un groupe alkyle en $C_{1-4}$ non substitué ou bien un groupe phényle non substitué, ou monosubstitué par un atome de chlore, le groupe méthyle ou méthoxy; R′ et R″ sont chacun le groupe méthyle ou éthyle ou bien forment ensemble le groupe $-(CH_2)_5-$, et R‴ est le groupe méthyle ou bien un groupe alkyle en $C_{2-6}$ non substitué ou substitué par un radical alcoxy en $C_{1-4}$ ou alcoxyalcoxy en $C_{3-6}$, en particulier un groupe alkyle en $C_{1-4}$ non substitué.

6. Composés de formule I selon la revendication 1, dans lesquels X est un atome de brome; $R_1$ est le groupe méthyle et en particulier un atome d'hydrogène; $R_2$ est un groupe phényle, $-CN$

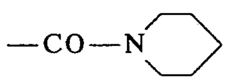

ou $-COOR'''$; R‴ est un groupe alkyle en $C_{1-4}$ non substitué, et $R_3$ est un atome d'hydrogène, le groupe méthyle, $-CH_2COOCH_3$ ou $-CH_2COOC_2H_5$, au moins un des symboles $R_1$ et $R_3$ étant de l'hydrogène.

7. Le composé selon la revendication 1, ayant la formule:

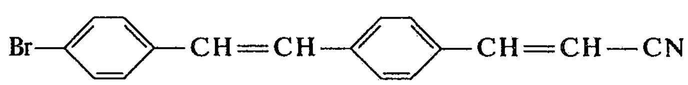

8. Le composé selon la revendication 1, ayant la formule:

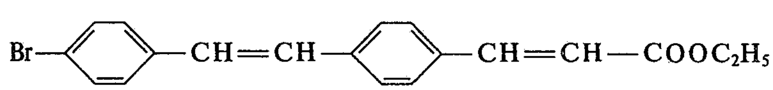

9. Procédé pour la préparation des composés de formule I selon la revendication 1, caractérisé par le fait qu'on fait réagir, soit:

a) un composé de formule IIa

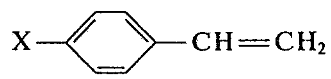

(IIa)

en présence d'une base et en ajoutant du palladium métallique ou bien des composés du palladium, qui forment dans les conditions réactionnelles des composés du palladium(O) labiles sans phosphore, comme catalyseur, avec un composé de formule IIIa

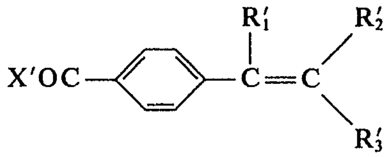

(IIIa)

soit

b) un composés de formule IIb

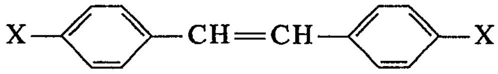

(IIb)

en présence d'une base et d'un composé du palladium arsénié ou phosphoré, comme catalyseur, avec un composé de formule IIIb

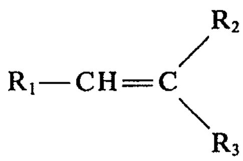

(IIIb)

X, $R_1$, $R_2$ et $R_3$ ayant les significations données dans la revendication 1 ; X' est un atome de chlore, de brome ou d'iode, et $R'_1$, $R'_2$ et $R'_3$ peuvent avoir la même signification que $R_1$, $R_2$ et $R_3$ mais sont exempts de substituant, qui sont capables de réagir avec les groupes X'OC—.

10. Procédé selon la revendication 9, caractérisé par le fait qu'on utilise un composé de formule IIIa, dans lequel X' est un atome de chlore.

11. Procédé selon la revendication 10, caractérisé par le fait qu'on utilise le 4-bromostyrène comme composé IIa.

12. Procédé selon la revendication 9, caractérisé par la fait qu'on utilise dans la variante opératoire a) comme composé du palladium $PdCl_2$, $PdBr_2$, $Pd(OOCCH_3)_2$, $Pd(CH_3COCHCOCH_3)_2$, $Pd(OOCCH_3)_2$(2,2'-bipyridyle), $PdCl_2(NC\text{-phényle})_2$, bis-(dibenzylidène-acétone)palladium(O) ou bis-(cyclohexylisonitrile)-palladium(O).

13. Procédé selon la revendication 9, caractérisé par le fait que dans la variante opératoire a) on utilise comme composés du palladium $PdCl_2$, l'acétate de palladium ou le bis-(dibenzylidène-acétone)palladium(O).

14. Procédé selon la revendication 9, caractérisé par le fait que dans la variante opératoire b), on utilise comme catalyseur un mélange d'acétate de palladium et de triphénylphosphine ou de tri-o-tolylphosphine.

15. Procédé selon la revendication 9, caractérisé par le fait qu'on utilise comme base la N-benzyldiméthylamine, la N-éthylmorpholine ou la tri-n-butylamine.

16. Procédé selon la revendication 9, caractérisé par le fait qu'on utilise le catalyseur à raison de 0,001 à 3% en mole par rapport aux composés de formule IIa ou IIb.

17. Les composés de formules:

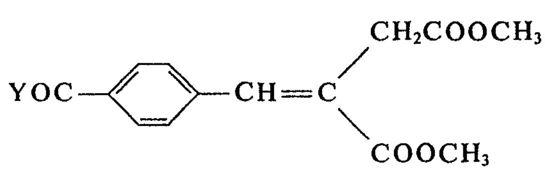

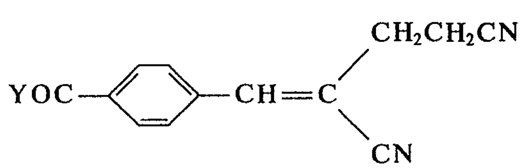

et

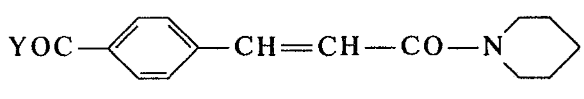

dans lesquels Y est un groupe —OH ou Cl.